COMPLETE DOCUMENT

(19) Europäisches Patentamt
European Patent Office
Office européen des brevets

(11) Numéro de publication: **0 101 380**
**A2**

(12) # DEMANDE DE BREVET EUROPEEN

(21) Numéro de dépôt: 83401638.8

(22) Date de dépôt: 10.08.83

(51) Int. Cl.³: **C 07 D 401/12**
C 07 D 213/89, A 61 K 31/44
//C07D211/46, C07D211/14, C07D211/22, C07D213/81, C07D213/64, C07D295/08, C07C91/30, C07C93/14

(30) Priorité: 13.08.82 FR 8214128
22.04.83 FR 8306681

(43) Date de publication de la demande: 22.02.84 Bulletin 84/8

(84) Etats contractants désignés: AT BE CH DE FR GB IT LI LU NL SE

(71) Demandeur: SANOFI, société anonyme
40, Avenue George V
F-75008 Paris(FR)

(72) Inventeur: Nisato, Dino
Viale Golgi, 80
Pavia(IT)

(72) Inventeur: Boveri, Sergio
Corso Republica 48
Tortona(IT)

(74) Mandataire: Combe, André et al,
CABINET BEAU DE LOMENIE 55 rue d'Amsterdam
F-75008 Paris(FR)

(54) **Nicotinamide 1-oxyde N-substitué, ses sels, procédé pour leur préparation et compositions pharmaceutiques en contenant.**

(57) Nicotinamide 1-oxyde N-substituté à action antagoniste des récepteurs $H_2$ de l'histamine, de formule

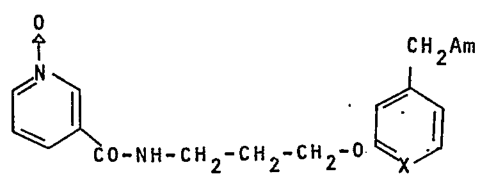

dans laquelle X est un atome d'azote ou un groupe CH et Am représente un groupe amino mono- ou disubstitué, un groupe pyrrolidino, morpholino, thiomorpholino, hexaméthylèneimino ou un groupe pipéridino ou pipérazino éventuellement substitués; leurs sels; un procédé pour leur préparation; et compositions pharmaceutiques les renfermant en tant que principes actifs.

EP 0 101 380 A2

Nicotinamide 1-oxyde N-substitué, ses sels, procédé pour leur préparation et compositions pharmaceutiques en contenant.

La présente invention concerne de nouveaux dérivés du nicotinamide 1-oxyde ayant une activité bloquant les récepteurs $H_2$ de l'histamine, leurs sels, un procédé pour leur préparation, ainsi que des compositions pharmaceutiques renfermant lesdits dérivés en tant qu'ingrédients actifs.

Plus particulièrement, la présente invention concerne, selon un des aspects, un nicotinamide 1-oxyde N-substitué de formule

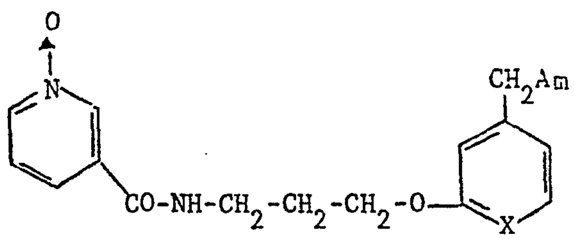

I

dans laquelle X représente un atome d'azote ou un groupe CH ; Am représente un groupe amino substitué par un alkyle inférieur, par un alkyl inférieur portant un groupe hydroxy, phényle ou cycloalkyle ayant 3 à 6 atomes de carbone ou par un radical choisi parmi alkényle inférieur, alkynyle inférieur, phényle et cycloalkyle ayant de 3 à 6 atomes de carbone ; un groupe amino substitué par deux groupes alkényle inférieur ou alkynyle inférieur, identiques ou différents ; un groupe amino substitué par deux groupes (hydroxy)alkyle inférieur ou (alkoxy inférieur)alkyle inférieur, identiques ou différents ; un groupe dialkylamino inférieur ; un groupe dialkylamino inférieur substitué par un groupe phényle, cycloalkyle ayant 3 à 6 atomes de carbone, méthoxyle ou hydroxyle ; un groupe pyrrolidino ; un groupe morpholino ; un groupe thiomorpholino ; un groupe hexaméthylèneimino ; un groupe pipéridino ; un groupe pipéridino substitué par un groupe méthyle, hydroxyle ou hydroxyméthyle ou par deux groupes méthyles ; un groupe pipérazino substitué en position 4 par un groupe alkyle inférieur ou (hydroxy)alkyle inférieur ; ou un groupe pipérazino substitué en position 4 par un groupe alkyle inférieur ou (hydroxy)alkyle inférieur et portant aussi un groupe méthyle en positions 3 et/ou 5 ainsi que ses sels pharmaceutiquement acceptables.

Le terme "dialkylamino inférieur", tel qu'utilisé ici, désigne le groupe $NH_2$ substitué par deux groupes alkyle inférieur, identiques ou différents.

Les termes "alkyle inférieur", "alkényle inférieur" et "alkynyle inférieur", tels qu'utilisés ici, désignent les radicaux hydrocarbonés aliphatiques, saturés ou contenant une double ou une triple liaison, ayant jusqu'à 4 atomes de carbone.

Le terme "alkoxy inférieur" désigne la fonction hydroxyle dont l'atome d'hydrogène est remplacé par un groupe alkyle inférieur, tel que défini ci-dessus.

Les termes "pyrrolidino", "pipéridino" et "pipérazino", tels qu'utilisés ici dans la description comme dans les revendications, remplacent la nomenclature recommandée par IUPAC pour les radicaux "1-pyrrolidinyl", "1-pipéridinyl" et, respectivement, "1-pipérazinyl". Le terme "hexaméthylèneimino" est utilisé ici pour désigner le radical "1H-hexahydroazépin-1-yl".

Après la subdivision des récepteurs de l'histamine en récepteurs $H_1$ (Ash et Schild, Br. J. Pharmacol. Chemother. 1966, 27, 427) et récepteurs $H_2$ (Black et al., Nature 1972, 236, 385) et la découverte que le blocage sélectif des récepteurs $H_2$ provoque une inhibition de la sécrétion gastrique, de nombreux produits ont été proposés comme antagonistes des récepteurs $H_2$ de l'histamine, ci-après indiqués "$H_2$-bloquants". Ainsi, les composés ayant reçu les Dénominations Communes Internationales burimamide, métiamide, cimétidine, ranitidine, tiotidine, étintidine, oxmétidine ont fait l'objet d'un grand nombre de publications scientifiques.

Tous les produits ci-dessus sont caractérisés par la présence dans leur molécule de la structure suivante :

$$-NH-\underset{\underset{Z}{\|}}{C}-NH- \qquad II$$

où Z représente un atome d'oxygène ou de soufre, ou bien un groupe N-CN, N-CO ou $CH-NO_2$, ladite structure étant linéaire et liée à deux groupements aliphatiques ou incluse dans un cycle comme dans le cas de l'oxmétidine.

La cimétidine, 2-cyano-1-méthyl-3-[2-[[(5-méthyl-imidazol-4-yl)méthyl]thio]éthyl]guanidine, comportant la structure II où Z est N-CN, et la ranitidine, N-[2-[[5-[(diméthylamino)méthyl]furfuryl]thio]éthyl]-N'-méthyl-2-nitro-1,1-éthènediamine, comportant la structure II où Z est $CH-NO_2$, sont déjà utilisées en thérapeutique pour le traitement de l'ulcère gastrique et duodénal.

La demande de brevet européen 23 578 décrit des dérivés de l'aminoalkylbenzène de formule

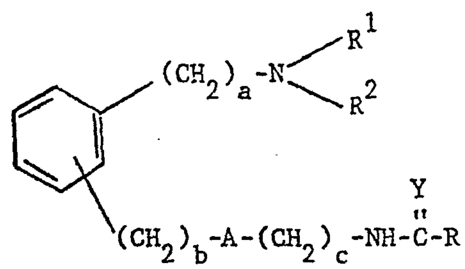

III

utiles comme $H_2$-bloquants.

Plus particulièrement, ladite demande de brevet indique que les composés de formule III, disubstitués en 1,3 sur le noyau benzène, où $NR^1R^2$ est pyrrolidino, a = 1, b = 0, A = oxygène, c = 3, Y = oxygène et R = hydrogène ou 3-pyridyle (composés IV et V ci-dessous), possèdent une DE50, respectivement de 0,84 et 1,25 mg/kg/i.v. dans le test de l'hypersécrétion gastrique selon Ghosh et Schild.

On a maintenant trouvé d'une manière surprenante que les composés de formule I ci-dessus, caractérisés par la présence d'un amide de l'acide nicotinique 1-oxyde, possèdent une activité $H_2$-bloquante extraordinairement élevée et de relativement longue durée.

La sélectivité de l'activité des produits de la présente invention vers les récepteurs du type $H_2$ est confirmée par l'absence d'activité du type $H_1$ dans le test de la contraction provoquée par l'histamine sur l'iléon isolé de cobaye.

L'activité antagoniste des composés de la présente invention vers les récepteurs $H_2$ gastriques de l'histamine a été confirmée dans le test de l'activité antisécrétoire basé sur l'antagonisme pour l'hypersécrétion provoquée par l'histamine chez le rat

atropinisé, selon la méthode de Ghosh et Schild (Br. J. Pharmacol. Chemother. 1958, 13, 54) modifiée selon Black (Nature 1972, 236, 385). Selon ce test, on provoque une hypersécrétion acide gastrique par infusion intraveineuse d'une dose submaximale d'histamine équivalant à 15 mcmol/kg.heure et l'on mesure la sécrétion gastrique par perfusion d'une solution physiologique à une vitesse constante dans l'estomac de l'animal.

Comme composés de référence, on a utilisé la cimétidine et la ranitidine, qui constituent les composés standards, ainsi que :

- le composé de formule

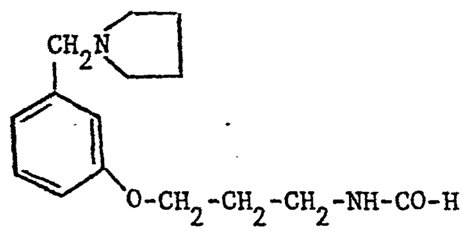

IV

sous forme d'oxalate et

- le composé de formule

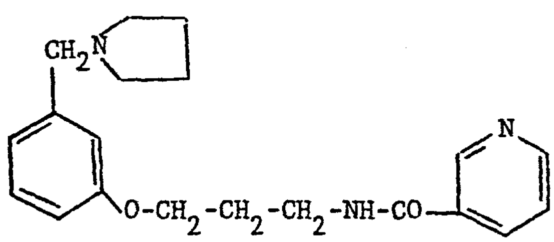

V

sous forme de dioxalate ;
décrits dans la demande de brevet européen 23 578.

Comme composés représentatifs de la présente invention, on a utilisé les composés des exemples 1, 2, 6 à 10 et 20 ci-dessous, désignés respectivement par leurs codes CM 57891, SR 58017A, SR 58062, SR 58037, SR 58042, SR 58067, SR 58052 et SR 58065.

Le tableau I montre, pour chaque produit, la dose (en mcmol/kg par voie veineuse en dose unique) qui inhibe de 50 % l'hypersécrétion gastrique provoquée par l'histamine (DI50) ainsi que la puissance relative par rapport à la cimétidine.

TABLEAU I

| Composé | DI50 (mcmol/kg) | Puissance relative (cimétidine = 1) |
|---|---|---|
| Cimétidine | 0,95 | 1,00 |
| Ranitidine | 0,25 | 3,80 |
| Composé IV | 2,01 | 0,47 |
| Composé V | 2,22 | 0,43 |
| CM 57891 | 0,39 | 2,43 |
| SR 58017A | 0,11 | 8,64 |
| SR 58062 | 0,80 | 1,19 |
| SR 58037 | 0,13 | 7,31 |
| SR 58042 | 0,14 | 6,78 |
| SR 58067 | 0,71 | 1,34 |
| SR 58052 | 0,25 | 3,80 |
| SR 58065 | 0,64 | 1,48 |

De ce tableau il ressort que les composés représentatifs de la présente invention ont une activité extrêmement élevée, qui, pour deux produits est presque double que celle de la ranitidine. Par rapport aux composés IV et V décrits dans la demande de brevet européen 23 578, les composés de la présente invention ont une activité antisécrétoire jusqu'à 20 fois supérieure.

Les composés de formule I ci-dessus, ainsi que leurs sels pharmaceutiquement acceptables, sont préparés, selon un autre aspect de la présente invention, suivant un procédé caractérisé en ce qu'on traite une amine de formule

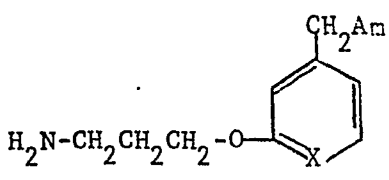

VI

dans laquelle X et Am sont tels que définis ci-dessus, avec un dérivé fonctionnel de l'acide nicotinique 1-oxyde de formule

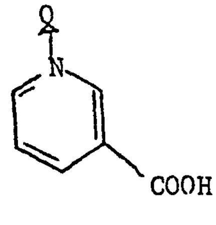

VII

dans un solvant organique à une température comprise entre 0°C et la température d'ébullition du solvant employé et l'on transforme éventuellement le produit ainsi obtenu dans ses sels pharmaceutiquement acceptables.

Comme dérivé fonctionnel approprié, on peut utiliser l'acide libre activé, l'anhydride, un anhydride mixte, le chlorure ou un ester actif.

Un dérivé fonctionnel convenable de l'acide de formule formule VII ci-dessus est le chlorure, sous forme de chlorhydrate de préférence, ou un ester actif de formule

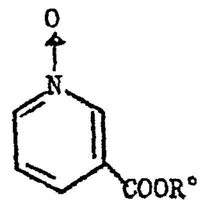

VIII

dans laquelle R° représente un groupe nitrophényle, méthoxyphényle, trityle, benzhydryle.

La température de réaction peut varier entre 0°C et la température d'ébullition du solvant employé, mais en général on opère à la température ambiante ou à 30-50°C. Il peut être préférable de conduire la réaction au froid lorsqu'elle est exothermique, par exemple lorsqu'on utilise le chlorure en tant que dérivé fonctionnel de l'acide carboxylique de formule VII.

Comme solvant de réaction, on utilise de préférence un alcool, tel que le méthanol, ou un solvant halogéné tel que le chlorure de méthylène, le dichloroéthane, le chloroforme et similaires, mais d'autres solvants organiques compatibles avec les réactifs employés, par exemple l'acétonitrile, le dioxane, le tétrahydrofuranne ou un hydrocarbure tel que l'hexane, peuvent être également employés.

La réaction peut être conduite en présence d'un accepteur de protons, par exemple d'un carbonate alcalin ou d'une amine tertiaire, lorsque de l'acide chlorhydrique, ou un autre acide, se libère pendant la réaction, mais cet accepteur de protons n'est pas indispensable pour l'obtention du produit final.

La réaction est assez rapide ; en général, après 1 à 4 heures à la température ambiante ou à 30-50°C, la réaction est achevée et le composé de formule I obtenu est isolé selon les techniques conventionnelles sous forme de base libre ou d'un de ses sels.

La base libre peut être transformée dans un de ses sels pharmaceutiquement acceptables par traitement avec une solution de l'acide convenable dans un solvant organique. Si le composé I est isolé sous forme de sel, la base libre correspondante peut être libérée à l'aide d'un hydroxyde ou d'un carbonate alcalin.

Les composés de départ de formule VI ci-dessus, où X est CH, sont connus en littérature ou ils peuvent être aisément préparés à partir du 3-hydroxybenzaldéhyde (IX) par amination réductive avec l'amine Am-H (X) en utilisant le borohydrure de sodium dans du méthanol comme agent de réduction et par réaction de l'aminophénol (XI) ainsi obtenu avec le chlorhydrate de 3-chloropropylamine en présence d'hydrure de sodium dans le diméthylformamide, selon le schéma suivant

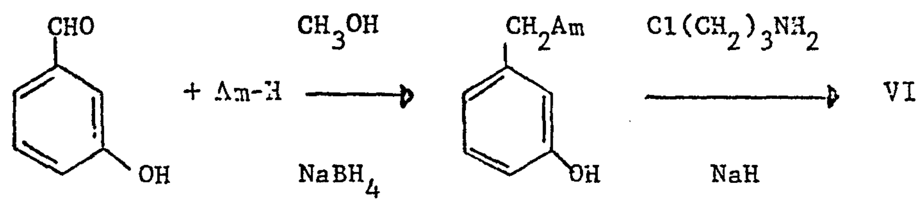

IX X XI

dans lequel Am est tel que défini ci-dessus.

Les composés de départ de formule VI ci-dessus, où X est N, sont connus ou ils peuvent être préparés à partir de l'acide 2-chloroisonicotinique (XII) en le transformant d'abord dans le chlorure d'acide par action du chlorure de thionyle et ensuite dans l'amide substitué (XIII) par réaction avec l'amine Am-H (X), en faisant réagir l'amide (XIII) ainsi obtenu avec le 3-hydroxypropyl-2,5-diméthylpyrrole, en soumettant l'amine bloquée (XIV) ainsi obtenue à une réduction avec l'hydrure de lithium et d'aluminium et en faisant réagir l'aminométhylpyridyloxypropylpyrrole (XV) ainsi obtenu avec le chlorhydrate d'hydroxylamine pour libérer l'amine VI, selon le schéma suivant

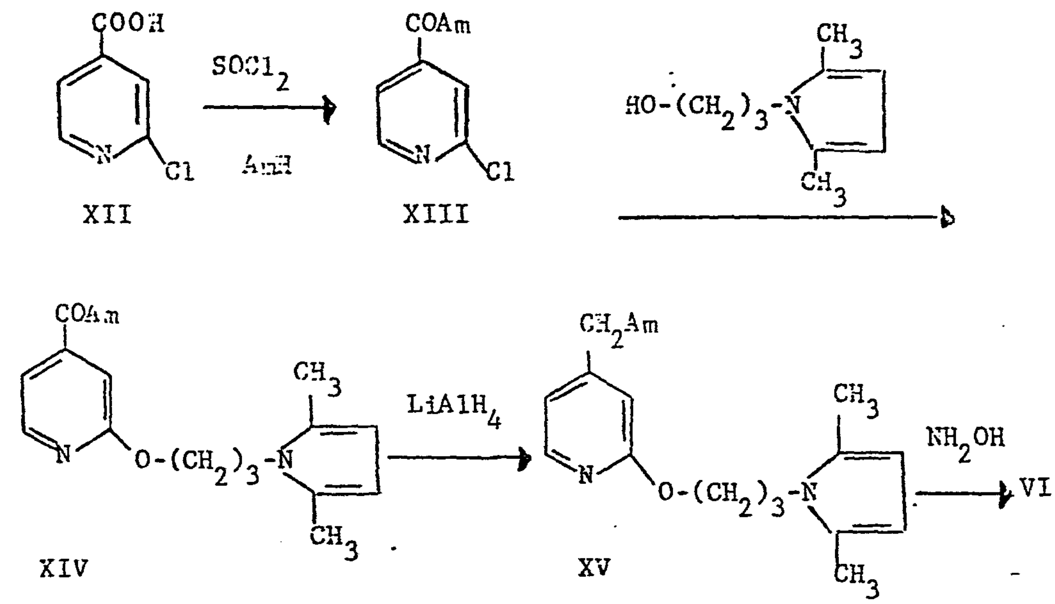


XII XIII XIV XV

Les composés de formule VIII ci-dessus sont connus en littérature ou bien ils peuvent être aisément préparés par réaction de l'acide VII avec l'alcool ou le phénol approprié en présence d'un agent de condensation tel que dicyclohexylcarbodiimide dans un solvant tel que le chlorure de méthylène.

Les composés de la présente invention sont peu toxiques et sont utiles comme médicaments.

Ainsi, selon un autre de ses aspects, la présente invention se réfère à des compositions pharmaceutiques renfermant, en tant qu'ingrédients actifs, les composés de formule I ci-dessus, ainsi que leurs sels d'addition pharmaceutiquement acceptables.

Dans les compositions pharmaceutiques de la présente invention pour l'administration orale, sublinguale, sous-cutanée, intramusculaire, intraveineuse, transdermique ou rectale, les ingrédients actifs de formule I ci-dessus peuvent être administrés sous des formes unitaires d'administration, en mélange avec des supports pharmaceutiques classiques, aux animaux et aux êtres humains pour le traitement de l'hypersécrétion gastrique et de la maladie ulcéreuse. Les formes unitaires d'administration appropriées comprennent les formes par voie orale telles que les comprimés, les gélules, les poudres, les granules et les solutions ou suspensions orales et les formes d'administration sublinguale et buccale, ainsi que les formes

d'administration parentérale utiles pour une injection sous-cutanée, intramusculaire ou intraveineuse, de même que les formes d'administration rectale.

Afin d'obtenir l'effet antisécrétoire désiré, la dose de principe actif peut varier entre 0,1 et 100 mg par kg de poids du corps et par jour.

Chaque dose unitaire peut contenir de 10 à 1 000 mg, de 50 à 500 mg de préférence, d'ingrédient actif en combinaison avec un support pharmaceutique. Cette dose unitaire peut être administrée 1 à 4 fois par jour pour traiter l'hypersécrétion gastrique et la maladie ulcéreuse.

Les exemples suivants illustrent l'invention sans toutefois la limiter.

PREPARATION 1.

a) 3-morpholinométhylphénol.

A une solution de 0,2 mol de 3-hydroxybenzaldéhyde dans 100 ml de méthanol, on ajoute, à environ 20°C, une solution de 0,4 mol de morpholine dans 40 ml de méthanol. Dans le mélange obtenu, on introduit par portions, sous agitation à la température de 20-25°C et en 90 minutes environ, 0,2 mol de borohydrure de sodium, puis on agite le mélange réactionnel 3 heures à la température ambiante et on évapore le solvant sous pression réduite jusqu'à siccité. On reprend le résidu par de la glace, on l'acidifie avec de l'acide chlorhydrique, on lave la solution acide 2 fois avec 40 ml d'acétate d'éthyle et on y ajoute de l'hydroxyde d'ammonium concentré jusqu'à réaction nettement basique. On extrait 4 fois avec 80 ml d'acétate d'éthyle et on évapore le solvant. Le produit ainsi obtenu fond à 113-116°C. Rendement : 67 %.

b) 3-(3-morpholinométhylphénoxy)propylamine

A une suspension de 0,28 mol d'hydrure de sodium à 80 % dans du diméthylformamide, on ajoute par portions, sous agitation à la température d'environ 20°C, 0,06 mol de 3-morpholinométhylphénol, puis on agite 15 minutes à la température ambiante et on ajoute ensuite au mélange, à 5-10°C, une solution de 0,08 mol de chlorhydrate de 3-chloropropylamine dans 20 ml de diméthylformamide. On agite le mélange réactionnel 24 heures à la température ambiante,

puis on y ajoute 400 ml d'eau glacée et on extrait 4 fois avec 100 ml d'acétate d'éthyle. On lave la solution organique 2 fois avec 30 ml d'hydroxyde de sodium à 10 % et ensuite 3 fois avec 20 ml d'eau. On sèche sur du sulfate de sodium anhydre et on évapore à sec sous pression réduite. On obtient ainsi le 3-(3-morpholinométhylphénoxy)propylamine sous forme d'une huile jaune. Rendement : 64 %.

PREPARATION 2.

3-(3-thiomorpholinométhylphénoxy)propylamine.

En opérant comme décrit ci-dessus, par réaction de 0,2 mol de 3-hydroxybenzaldéhyde avec 0,4 mol de thiomorpholine en présence de 0,2 mol de borohydrure de sodium (comme dans la PREPARATION 1a), on obtient le 3-thiomorpholinométhylphénol qui, traité avec un excès de chlorhydrate de 3-chloropropylamine en présence d'hydrure de sodium (comme dans la PREPARATION 1b), donne la 3-(3-thiomorpholinométhylphénoxy)propylamine, sous forme d'huile.

PREPARATION 3.

a) 3-(4-hydroxypipéridinométhyl)phénol.

A une solution de 0,2 mol de 3-hydroxybenzaldéhyde dans 100 ml de méthanol, on ajoute, à environ 20°C, une solution de 0,4 mol de 4-hydroxypipéridine dans 80 ml de méthanol. Dans le mélange obtenu, on introduit, par portions et sous agitation, à la température de 20-25°C et en 90 minutes environ, 0,2 mol de borohydrure de sodium, puis on agite le mélange réactionnel 4 heures à la température ambiante et on évapore le solvant sous pression réduite jusqu'à siccité. On reprend le résidu par de la glace, on acidifie avec de l'acide chlorhydrique, on lave la solution acide 2 fois avec 40 ml d'acétate d'éthyle et on y ajoute de l'hydroxyde d'ammonium concentré jusqu'à réaction nettement basique. On filtre le produit précipité et on le cristallise de 400 ml d'eau. Le produit ainsi obtenu fond à 156-158°C. Rendement : 66,8 %.

b) 3-[3-(4-hydroxypipéridinométhyl)phénoxy]propylamine.

A une suspension de 0,36 mol d'hydrure de sodium à 50 % dans 180 ml de diméthylformamide, on ajoute par portions sous agitation, à la température d'environ 20°C, 0,14 mol de 3-(4-hydroxypipéridinométhyl)phénol, puis on agite 15 minutes à 40°C et, ensuite, on ajoute au mélange, à 5-10°C, une solution de 0,18 mol de chlorhydrate de 3-chloropropylamine dans 80 ml de diméthylformamide. On agite le mélange réactionnel 24 heures à la température ambiante, puis on y ajoute 400 ml d'eau glacée contenant de l'acide chlorhydrique et on ajuste le pH de la solution à 6. On lave la solution obtenue 2 fois avec 100 ml d'acétate d'éthyle et on l'alcalinise avec de l'hydroxyde de sodium concentré. On extrait 4 fois avec 80 ml d'acétate d'éthyle, on sèche la phase organique sur du sulfate de sodium anhydre et on évapore à sec sous pression réduite. On chromatographie l'huile obtenue avec du méthanol et, par évaporation de la fraction pure, on isole le produit sous forme d'une huile jaune. Rendement : 16 %.

PREPARATIONS 4 à 13.

En opérant comme décrit ci-dessus, par réaction de 0,2 mol de 3-hydroxybenzaldéhyde avec 0,4 mol de, respectivement, pipéridine, 4-méthylpipéridine, 3-méthylpipéridine, hexaméthylèneimine, méthylbenzylamine, 3-hydroxyméthylpipéridine, 4-méthylpipérazine, diméthylamine, di-n-propylamine, di-n-butylamine en présence de 0,2 mol de borohydrure de sodium (comme dans la PREPARATION 3a), on obtient les aminométhylphénols correspondants qui, traités avec un excès de chlorhydrate de 3-chloropropylamine en présence d'hydrure de sodium (comme dans la PREPARATION 3b), donnent les composés correspondants de formule VI ci-dessus.

Les caractéristiques des aminométhylphénols XI et des intermédiaires VI ainsi obtenus sont résumées dans le tableau II.

TABLEAU II

| Préparation | Am | Caractéristiques Physico-chimiques | |
|---|---|---|---|
| | | Composé XI | Composé VI |
| 4 | 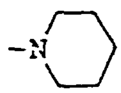 | F. 128-130°C | F. 202-205°C (dichlorhydrate) |
| 5 | 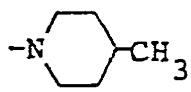 | huile jaune-pâle | F. 148-151°C (dioxalate) |
| 6 | 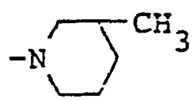 | F. 155-157°C | huile jaune |
| 7 | 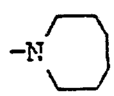 | F. 116-118°C | huile jaune |
| 8 | 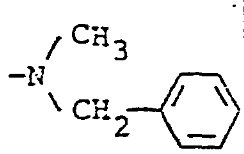 | F. 206-211°C (chlorhydrate) | F. 147-149°C (chlorhydrate) |
| 9 | 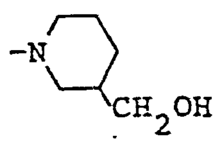 | huile | huile |
| 10 | 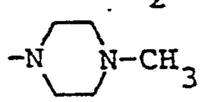 | F. 255-258°C (chlorhydrate) | F. 253-255°C (trichlorhydrate) |
| 11 | 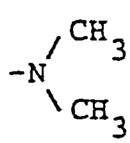 | F. 106-109°C | F. 213-216°C (dichlorhydrate) |
| 12 | 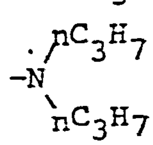 | F. 184-186°C (chlorhydrate) | F. 204-206°C (dichlorhydrate) |
| 13 | 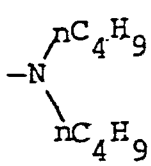 | F. 50-55°C | huile |

PREPARATION 14.

a) 2-chloro-4-pipéridinocarbonylpyridine.

On chauffe au reflux pendant 2 heures un mélange de 0,13 mol d'acide 2-chloroisonicotinique et 70 ml de chlorure de thionyle, puis on concentre sous pression réduite et on reprend le résidu avec du toluène anhydre. On évapore le solvant et l'on suspend le chlorure de l'acide 2-chloroisonicotinique brut, ainsi obtenu sous forme d'un solide jaune, dans 50 ml de chlorure de méthylène. On refroidit le mélange à 0-5°C et on y ajoute lentement une solution de 0,3 mol de pipéridine dans 50 ml de chlorure de méthylène. On agite 2 heures à la température ambiante, on lave à fond à l'eau et l'on évapore à sec la phase organique. On obtient ainsi 19 g de 2-chloro-4-pipéridinocarbonylpyridine qui est purifiée par chromatographie sur gel de silice en éluant avec de l'acétate d'éthyle. On obtient ainsi 10 g de produit pur sous forme d'une huile jaune.

b) 2-[3-(2,5-diméthylpyrrol-1-yl)propoxy]-4-pipéridino-carbonylpyridine.

A une suspension de 0,047 mol de 2,5-diméthyl-1-(3-hydroxypropyl)pyrrole et 0,059 mol d'hydrure de sodium à 80 % dans 150 ml de 1,2-diméthoxyéthane, chauffée 15 minutes au reflux au préalable et refroidie, on ajoute 0,044 mol de 2-chloro-4-pipéridinocarbonylpyridine, on chauffe ensuite le mélange réactionnel 2 heures au reflux et on l'évapore à sec. On reprend le résidu avec de l'eau, on extrait avec de l'acétate d'éthyle, on sépare la phase organique et l'on évapore à sec. On obtient ainsi 15,4 g de produit huileux.

c) 2-[3-(2,5-diméthylpyrrol-1-yl)propoxy]-4-pipéridino-méthylpyridine.

On chauffe au reflux pendant 1 heure un mélange de 15,4 g du produit ainsi obtenu, 2 g d'hydrure de lithium et d'aluminium et 150 ml de 1,2-diméthoxyéthane, puis on refroidit et on ajoute lentement de l'eau au mélange. On filtre, on évapore à sec la solution obtenue, on reprend le résidu avec de l'eau et l'on acidifie avec de l'acide chlorhydrique N. On lave la solution 2 fois avec 30 ml d'acétate d'éthyle, on ajoute de l'hydroxyde de sodium

jusqu'à réaction basique et l'on extrait 4 fois avec 30 ml d'acétate d'éthyle. On sèche la phase organique sur du sulfate de sodium anhydre et l'on évapore à sec. On obtient ainsi 8,7 g de produit huileux.

d) 3-(4-pipéridinométhylpyrid-2-yloxy)propylamine.

A une solution de 8,6 g de chlorhydrate d'hydroxylamine dans 26 ml d'éthanol, sous agitation à la température ambiante depuis 30 minutes, on ajoute une solution de 2,8 g d'hydroxyde de potassium à 85 % dans 10 ml d'éthanol à 50 %, puis on agite encore 10 minutes et on ajoute 8,7 g de 2-[3-(2,5-diméthylpyrrol-1-yl)propoxy]-4-pipéridinométhyl-pyridine. On chauffe au reflux pendant 6 heures, puis on filtre, on lave avec de l'éthanol, on acidifie par de l'acide chlorhydrique N et l'on lave 2 fois avec 30 ml d'acétate d'éthyle. On ajuste le pH de la solution aqueuse à 3, on lave encore 2 fois avec 30 ml d'acétate d'éthyle et l'on rend la solution nettement basique par addition d'une solution concentrée d'hydroxyde de potassium. On extrait avec de l'acétate d'éthyle, on sèche la phase organique sur du sulfate de sodium anhydre et évapore. On obtient ainsi 5,3 g de 3-(4-pipéridinométhylpyrid-2-yloxy)propylamine sous forme d'huile.

PREPARATION 15.

On traite l'acide 2-chloroisonicotinique avec le chlorure de thionyle et, ensuite, le chlorure d'acide ainsi obtenu avec la diméthylamine (comme décrit dans la PREPARATION 14a), pour obtenir le diméthylamide de l'acide 2-chloroisonicotinique qui, par condensation avec 2,5-diméthyl-1-(3-hydroxypropyl)pyrrole en présence d'hydrure de sodium (comme décrit dans la PREPARATION 14b), donne le N,N-diméthyl-2-[3-(2,5-diméthylpyrrol-1-yl)propoxy]isonicotinamide (huile). On réduit le produit ainsi obtenu avec l'hydrure de lithium et d'aluminium (comme décrit dans la PREPARATION 14c) et l'on débloque la 2-[3-(2,5-diméthylpyrrol-1-yl)propoxy]-4-diméthylaminométhylpyridine par action de l'hydroxylamine (comme décrit dans la PREPARATION 14d) et on isole la 3-(4-diméthylaminométhylpyrid-2-yloxy)propylamine sous forme d'une huile.

PREPARATIONS 16 à 33.

En opérant comme décrit dans la PREPARATION 1, par réaction de 0,2 mol de 3-hydroxybenzaldéhyde avec 0,2 mol de l'amine appropriée en présence de 0,2 mol de borohydrure de sodium et par traitement du produit ainsi obtenu avec le chlorhydrate de 3-chloropropylamine en présence d'hydrure de sodium, on obtient :

- la 3-(3-butylaminométhylphénoxy)propylamine (16),
- la 3-(3-allylaminométhylphénoxy)propylamine (17),
- la 3-(3-propargylaminométhylphénoxy)propylamine (18),
- la 3-[(2-hydroxyéthyl)aminométhylphénoxy]propylamine (19),
- la 3-(3-benzylaminométhylphénoxy)propylamine (20),
- la 3-(3-cyclopropylméthylaminométhylphénoxy)propylamine (21),
- la 3-(3-phénylaminométhylphénoxy)propylamine (22),
- la 3-(3-cyclopentylaminométhylphénoxy)propylamine (23),
- la 3-(3-diallylaminométhylphénoxy)propylamine (24),
- la 3-(3-dipropargylaminométhylphénoxy)propylamine (25),
- la 3-[3-di(2-hydroxyéthyl)aminométhylphénoxy]propylamine (26),
- la 3-[3-di(2-méthoxyéthyl)aminométhylphénoxy]propylamine (27),
- la 3-[(méthyl-cyclopropylméthyl)aminométhylphénoxy]propylamine (28),
- la 3-[4-(2-hydroxyéthylpipérazino)méthylphénoxy]propylamine (29),
- la 3-(3,4,5-triméthylpipérazinométhylphénoxy)propylamine (30),
- la 3-(3,4-diméthylpipérazinométhylphénoxy)propylamine (31),
- la 3-(3,5-diméthylpipéridinométhylphénoxy)propylamine (32) et, respectivement,
- la 3-[4-(2-hydroxyéthyl)-3-méthylpipérazinophénoxy]propylamine (33).

EXEMPLE 1

A un mélange de 0,03 mol de 3-(3-pyrrolidinométhylphénoxy)propylamine et 0,095 mol de pyridine dans 100 ml de chlorure de méthylène, agité à 0°C, on ajoute, par portions, 0,047 mol de chlorhydrate du chlorure de l'acide nicotinique 1-oxyde, puis on agite une heure à la température ambiante et l'on évapore le solvant sous pression réduite. On reprend le résidu par de l'acide chlorhydrique N et on élimine par filtration les sels obtenus, puis on extrait 3 fois avec 100 ml d'acétate d'éthyle et on ajoute de l'hydroxyde de sodium jusqu'à pH basique. On extrait 4 fois avec 100 ml d'acétate d'éthyle, on sèche la phase organique sur du sulfate de

sodium anhydre et on évapore le solvant sous pression réduite. On chromatographie sur silice l'huile obtenue, en éluant avec du méthanol, on réunit les fractions de produit pur, l'on traite la solution ainsi obtenue avec une solution d'acide oxalique dans de l'éthanol et on cristallise de l'éthanol le précipité qui s'est formé. On obtient ainsi 3,5 g de sesquioxalate de N-[3-(3-pyrrolidinométhylphénoxy)propyl]-3-pyridinecarboxamide 1-oxyde, CM 57891A ; F. 115-116°C. Rendement : 30 % de la théorie.

Analyse pour $C_{20}H_{25}N_3O_3$, 1,5 $C_2H_2O_4$ :

| | C (%) | H (%) | N (%) |
|---|---|---|---|
| Calculé : | 56,32 | 5,75 | 8,57 |
| Trouvé : | 55,98 | 5,58 | 8,38 |

On met en suspension 1 g du produit ainsi obtenu dans de l'hydroxyde de sodium concentré, puis on extrait avec de l'acétate d'éthyle et on évapore la solution organique sous pression réduite jusqu'à siccité. Le résidu cristallise après 24-28 heures pour donner le N-[3-(3-pyrrolidinométhylphénoxy)propyl]-3-pyridinecarboxamide 1-oxyde, CM 57891 ; F. 101-103°C. Rendement : 90 % de la théorie.

Analyse pour $C_{20}H_{25}N_3O_3$ :

| | C (%) | H (%) | N (%) |
|---|---|---|---|
| Calculé : | 67,58 | 7,09 | 11,82 |
| Trouvé : | 67,01 | 7,02 | 11,68 |

<u>EXEMPLES 2 à 6</u>

En opérant comme décrit à l'Exemple 1, par réaction de 0,03 mol de 3-(3-pipéridinométhylphénoxy)propylamine, de 3-(3-morpholinométhylphénoxy)propylamine, de 3-(3-diméthylaminométhylphénoxy)propylamine, de 3-(3-di-n-propylaminométhylphénoxy)propylamine et de 3-(3-di-n-butylaminométhylphénoxy)propylamine avec 0,047 mol de chlorhydrate du chlorure de l'acide nicotinique 1-oxyde dans du chlorure de méthylène, on obtient, respectivement :

- le sesquioxalate de N-[3-(3-pipéridinométhylphénoxy)-propyl]-3-pyridinecarboxamide 1-oxyde, SR 58017A ; F. 115-118°C (Exemple 2) ; Rendement : 30 % de la théorie.

Analyse pour $C_{21}H_{27}N_3O_3$, 1,5 $C_2H_2O_4$ :

| | C (%) | H (%) | N (%) |
|---|---|---|---|
| Calculé : | 57,13 | 5,99 | 8,33 |
| Trouvé : | 57,00 | 5,93 | 8,27 |

- le sesquioxalate de N-[3-(3-morpholinométhylphénoxy)-propyl]-3-pyridinecarboxamide 1-oxyde, SR 58016A ; F. 152-155°C (Exemple 3) ; Rendement : 32 % de la théorie.

Analyse pour $C_{20}H_{25}N_3O_4$, 1,5 $C_2H_2O_4$ :

| | C (%) | H (%) | N (%) |
|---|---|---|---|
| Calculé : | 54,54 | 5,57 | 8,30 |
| Trouvé : | 54,26 | 5,66 | 8,18 |

- l'oxalate de N-[3-(3-diméthylaminométhylphénoxy)propyl]-3-pyridinecarboxamide 1-oxyde, SR 58059A ; F. 148-151°C (Exemple 4) ; Rendement : 35 % de la théorie.

Analyse pour $C_{18}H_{23}N_3O_3$, $C_2H_2O_4$ :

| | C (%) | H (%) | N (%) |
|---|---|---|---|
| Calculé : | 57,27 | 6,00 | 10,02 |
| Trouvé : | 56,83 | 6,04 | 9,97 |

- l'oxalate de N-[3-(3-di-n-propylaminométhylphénoxy)-propyl]-3-pyridinecarboxamide 1-oxyde, SR 58030A ; F. 168-170°C (Exemple 5) ; Rendement : 30 % de la théorie.

Analyse pour $C_{22}H_{31}N_3O_3$, $C_2H_2O_4$ :

| | C (%) | H (%) | N (%) |
|---|---|---|---|
| Calculé : | 60,62 | 6,92 | 8,84 |
| Trouvé : | 60,83 | 7,13 | 8,91 |

et

- le N-[3-(3-di-n-butylaminométhylphénoxy)propyl]-3-pyridinecarboxamide 1-oxyde, SR 58062 ; huile (Exemple 6) ; Rendement : 33 % de la théorie.

Analyse pour $C_{24}H_{35}N_3O_3$ :

| | C (%) | H (%) | N (%) |
|---|---|---|---|
| Calculé : | 69,70 | 8,53 | 10,16 |
| Trouvé : | 69,20 | 8,10 | 9,90 |

EXEMPLE 7

A un mélange de 0,023 mol de 3-[3-(4-méthylpipéridinométhyl)phénoxy]propylamine et 30 ml de pyridine, agité à 0°C, on ajoute, par portions, 0,047 mol de chlorhydrate du chlorure de

l'acide nicotinique 1-oxyde, puis on agite une heure à la température ambiante et l'on évapore le solvant sous pression réduite. On reprend le résidu par de l'acide chlorhydrique N et on élimine par filtration les sels obtenus, puis on extrait 3 fois avec 100 ml d'acétate d'éthyle et on ajoute de l'hydroxyde de sodium jusqu'à pH basique. On extrait avec de l'acétate d'éthyle contenant 10 % d'éthanol, on sèche la phase organique sur du sulfate de sodium anhydre et on évapore le solvant sous pression réduite. On obtient une huile qui cristallise lentement. On triture le produit dans de l'éther diéthylique et on le filtre. On obtient 4,5 g de produit brut qui est chromatographié sur silice dans un mélange méthanol : chloroforme 15 : 35 et cristallisé de l'acétate d'éthyle. On obtient ainsi 2,7 g de N-[3-[3-(4-méthylpipéridinométhyl)-phénoxy]propyl]-3-pyridinecarboxamide 1-oxyde, SR 58037, F. 109-111°C. Rendement : 35 % de la théorie.

Analyse pour $C_{22}H_{29}N_3O_3$ :

| | C (%) | H (%) | N (%) |
|---|---|---|---|
| Calculé : | 68,90 | 7,62 | 10,96 |
| Trouvé : | 68,69 | 7,85 | 11,11 |

EXEMPLES 8 et 9.

En opérant comme décrit dans l'exemple 7, par réaction de 0,03 mol de 3-[3-(3-méthylpipéridinométhyl)phénoxy]-propylamine et de 3-[3-(4-hydroxypipéridinométhyl)phénoxy]-propylamine avec 0,047 mol de chlorhydrate du chlorure de l'acide nicotinique 1-oxyde on obtient, respectivement :

- le N-[3-[3-(3-méthylpipéridinométhyl)phénoxy]propyl]-3-pyridinecarboxamide 1-oxyde, SR 58042 ; F. 108-110°C (Exemple 8) ; Rendement : 35 % de la théorie.

Analyse pour $C_{22}H_{29}N_3O_3$ :

| | C (%) | H (%) | N (%) |
|---|---|---|---|
| Calculé : | 68,90 | 7,62 | 10,96 |
| Trouvé : | 68,72 | 7,81 | 10,91 |

et

- le N-[3-[3-(4-hydroxypipéridinométhyl)phénoxy]propyl]-3-pyridinecarboxamide 1-oxyde, SR 58067 ; F. 113-116°C (Exemple 9) ; Rendement : 30 % de la théorie.

Analyse pour $C_{21}H_{26}N_3O_4$ :

| | C (%) | H (%) | N (%) |
|---|---|---|---|
| Calculé : | 65,44 | 7,06 | 10,90 |
| Trouvé : | 65,14 | 7,11 | 10,77 |

EXEMPLE 10.

A un mélange de 0,03 mol de 3-(3-hexaméthylèneiminométhylphénoxy)propylamine et 0,095 mol de pyridine dans 100 ml de chlorure de méthylène, agité à 0°C, on ajoute, par portions, 0,047 mol de chlorhydrate du chlorure de l'acide nicotinique 1-oxyde, puis on agite 90 minutes à la température ambiante et l'on évapore le solvant sous pression réduite. En opérant ensuite comme décrit à l'exemple 7, on obtient le N-[3-(3-hexaméthylèneiminométhylphénoxy) propyl]-3-pyridinecarboxamide 1-oxyde, SR 58052 ; F. 91-93°C. Rendement : 33 % de la théorie.

Analyse pour $C_{22}H_{29}N_3O_3$ :

| | C (%) | H (%) | N (%) |
|---|---|---|---|
| Calculé : | 68,90 | 7,62 | 10,95 |
| Trouvé : | 69,00 | 7,89 | 11,06 |

EXEMPLES 11 à 13.

En opérant comme décrit à l'Exemple 7, par réaction de 0,03 mol de 3-(3-benzylméthylaminométhylphénoxy)propylamine, de 3-[3-(3-hydroxyméthyl)pipéridinométhylphénoxy]propylamine et de 3-[3-(4-méthylpipérazinométhyl)phénoxy]propylamine avec 0,047 mol de chlorhydrate du chlorure de l'acide nicotinique 1-oxyde dans du chlorure de méthylène, on obtient, respectivement :

- le N-[3-(3-benzylméthylaminométhylphénoxy)propyl]-3-pyridinecarboxamide 1-oxyde, SR 58083 ; F. 94-96°C (Exemple 11) ; Rendement : 30 % de la théorie.

Analyse pour $C_{24}H_{27}N_3O_3$ :

| | C (%) | H (%) | N (%) |
|---|---|---|---|
| Calculé : | 71,09 | 6,71 | 10,36 |
| Trouvé : | 70,87 | 6,75 | 10,19 |

- le N-[3-[3-(3-hydroxyméthyl)pipéridinométhylphénoxy]-propyl]3-pyridinecarboxamide 1-oxyde, SR 58087 ; sous forme de solide vitreux (Exemple 12) ; Rendement : 20 % de la théorie.

Analyse pour $C_{25}H_{29}N_3O_4$ :

| | C (%) | H (%) | N (%) |
|---|---|---|---|
| Calculé : | 68,94 | 6,71 | 9,65 |
| Trouvé : | 68,56 | 6,81 | 9,47 |

$^1$HRMN (DMSO-d6) : 3,2 ppm (m, $CH_2OH$) ; 4,0 ppm (~t, $ArOCH_2$) ; 8,55 ppm (~t, 2 CH pyridine) ;

et

- le N-[3-[3-(4-méthylpipérazinométhyl)phénoxy]propyl]-3-pyridinecarboxamide 1-oxyde, sous forme de trichlorhydrate, SR 58084A ; F. 200-205°C (Exemple 13) ; Rendement : 45 % de la théorie,

Analyse pour $C_{21}H_{28}N_4O_3$, 3 HCl :

| | C (%) | H (%) | N (%) |
|---|---|---|---|
| Calculé : | 51,07 | 6,33 | 8,51 |
| Trouvé : | 50,85 | 6,35 | 8,44 |

EXEMPLE 14

A une solution de 0,04 mol de 3-[3-(3-méthylpipéridinométhyl)phénoxy]propylamine dans 40 ml d'acétonitrile on ajoute 0,04 mol de l'anhydre de l'acide nicotinique 1-oxyde (Synthesis, 1981, 618), puis on agite le mélange réactionnel 2 heures à 50-60°C et on l'évapore à sec sous pression réduite. On reprend le résidu avec 20 ml d'hydroxyde de sodium concentré et l'on extrait le produit 4 fois avec 30 ml d'acétate d'éthyle. On sèche la phase organique sur du sulfate de sodium anhydre, on évapore le solvant sous pression réduite et l'on purifie le produit ainsi obtenu par chromatographie sur silice comme décrit à l'exemple 7. On obtient ainsi le N-[3-[3-(3-méthylpipéridinométhyl)phénoxy]propyl]-3-pyridinecarboxamide 1-oxyde, identique au produit de l'exemple 8 ; F. 108-110°C.

EXEMPLE 15

A une solution de 0,03 mol d'acide nicotinique 1-oxyde dans 40 ml de chlorure de méthylène contenant 10 ml de triéthylamine on ajoute, à la température de 0-5°C et durant 10 minutes environ, 0,03 mol de chloroformiate d'isobutyle dissous dans 15 ml de chlorure de méthylène, puis on agite le mélange 30 minutes à la même température. On ajoute au même mélange et à la même température 0,03 mol de 3-[3-(3-méthylpipéridinométhyl)phénoxy]propylamine dissoute dans 15 ml de chlorure de méthylène, on agite 2 heures à la température ambiante puis on lave le mélange réactionnel avec 10 ml d'eau. On filtre la phase organique, on la sèche sur du sulfate de sodium anhydre, on l'évapore à sec sous pression réduite et on purifie le résidu par chromatographie sur silice comme décrit dans l'exemple 7. On obtient ainsi le N-[3-[3-(3-méthylpipéridinométhyl)phénoxy]propyl]-3-pyridinecarboxamide 1-oxyde, identique au produit de l'exemple 8.

EXEMPLE 16.

A une solution de 11,6 g d'acide nicotinique 1-oxyde, 11,2 g de 4-nitrophénol et 8,2 g de triéthylamine dans 160 ml de chlorure de méthylène on ajoute 17,6 g d'hexafluorophosphate de benzotriazolyloxy-tris-(diméthylamino)phosphonium. La température du mélange augmente et l'on obtient une solution d'où se sépare l'ester actif. On agite le mélange encore 30 minutes, puis on filtre le précipité et on le lave d'abord avec du chlorure de méthylène et après avec de l'éther diéthylique. On obtient ainsi 4 g de 3-pyridinecarboxylate de 4-nitrophényle 1-oxyde ; F. 234-236°C (déc.).

On ajoute l'ester actif ainsi obtenu à une solution de 3,8 g de 3-[3-(3-méthylpipéridinométhyl)phénoxy]propylamine dans 70 ml de méthanol et on agite le mélange 3 heures à 50°C. On évapore le solvant, on reprend le résidu avec 30 ml d'acide chlorhydrique N et l'on lave la solution acide 3 fois avec 20 ml d'acétate d'éthyle. On ajuste le pH à 7,8, on relargue avec du chlorure de sodium et l'on extrait 6 fois avec 30 ml d'acétate d'éthyle. On sèche les extraits organiques réunis sur du sulfate de sodium anhydre et on évapore le solvant à sec. On purifie le produit par chromatographie sur silice comme décrit à l'exemple 7. On obtient ainsi le N-[3-[3-(3-méthylpipéridinométhyl)-phénoxy]propyl]-3-pyridinecarboxamide 1-oxyde, identique au produit de l'exemple 8.

EXEMPLE 17.

A une solution de 5 g de 4-diméthylaminopyridine en 80 ml d'acétonitrile on ajoute 5,6 g d'acide nicotinique 1-oxyde. On agite le mélange 10 minutes à la température ambiante, puis on y ajoute 9,2 g de dicyclohexylcarbodiimide. Au bout de 5 minutes à la

température ambiante, on ajoute 10 g de 3-[3-(méthylpipéridinométhyl) phénoxy]propylamine dissoute dans 20 ml d'acétonitrile. On note une réaction faiblement exothermique (de 20 à 25°C) et une dissolution qui, au bout de 5 minutes, est presque complète. On agite ensuite le mélange 4 heures à 40°C, on filtre la dicyclohexylurée qui s'est formée et on la lave avec de l'acétonitrile. On évapore à sec sous pression réduite et l'on purifie le résidu par chromatographie sur silice comme décrit dans l' exemple 7. On obtient ainsi le N-[3-[3-(méthylpipéridinométhyl)phénoxy]propyl]-3-pipéridinecarboxamide 1-oxyde, identique au produit de l'exemple 8 ; F. 108-110°C.

EXEMPLE 18.

A une suspension de 2,8 g d'acide nicotinique 1-oxyde, 2,5 g de diméthylaminopyridine, 8,8 g d'hexafluorophosphate de benzotriazolyloxy-tris-(diméthylamino)phosphonium dans 40 ml d'acétonitrile on ajoute, à 20°C et sous agitation, 5 g de 3-[3-(3-méthylpipéridinométhyl)phénoxy]propylamine dissoute dans 10 ml d'acétonitrile. On note une élévation de la température jusqu'à 30°C et l'obtention d'une solution qui est ensuite agitée 2 heures à la température ambiante. On concentre le mélange sous pression réduite, on traite le résidu avec 20 ml d'hydroxyde de sodium concentré et on l'extrait 4 fois avec 40 ml d'acétate d'éthyle. Après l'évaporation des extraits organiques, on purifie le résidu par chromatographie sur silice comme décrit dans l'exemple 7. On obtient ainsi le N-[3-[3-(3-méthylpipéridinométhyl)phénoxy]propyl]-3-pyridinecarboxamide 1-oxyde, identique au produit de l'exemple 8.

EXEMPLE 19.

A un mélange de 0,03 mol de 3-(4-diméthylaminométhylpyrid-2-yloxy)propylamine et 0,095 mol de pyridine dans 100 ml de chlorure de méthylène, agité à 0°C, on ajoute, par portions, 0,047 mol de chlorhydrate du chlorure de l'acide nicotinique 1-oxyde, puis on agite 90 minutes à la température ambiante et l'on évapore le solvant sous pression réduite. En opérant ensuite comme décrit à l'exemple 1, on obtient le sesquioxalate de N-[3-(4-diméthylaminométhylpyrid-2-yloxy)propyl]-3-pyridinecarboxamide 1-oxyde.

Par neutralisation du produit ainsi obtenu avec de l'hydroxyde de sodium, on obtient le N-[3-(4-diméthylaminométhyl-

méthylpyrid-2-yloxy)propyl]-3-pyridinecarboxamide 1-oxyde, SR 58098 ; F. 103-106°C.

Analyse pour $C_{17}H_{22}N_4O_3$ :

| | C (%) | H (%) | N (%) |
|---|---|---|---|
| Calculé : | 61,80 | 6,71 | 17,04 |
| Trouvé : | 61,21 | 6,78 | 16,97 |

EXEMPLES 20 et 21.

En opérant comme décrit dans l'exemple 7, par réaction de 0,03 mol de 3-(4-pipéridinométhylpyrid-2-yloxy)propylamine et de 3-(4-diméthylaminométhylpyrid-2-yloxy)propylamine avec 0,047 mol de chlorhydrate du chlorure de l'acide nicotinique 1-oxyde on obtient, respectivement,

- le N-[3-(4-pipéridinométhylpyrid-2-yloxy)propyl]3-pyridinecarboxamide 1-oxyde, SR 58065 ; F. 87-89°C (Exemple 20) ; Rendement : 35 % de la théorie.

Analyse pour $C_{20}H_{26}N_4O_3$ :

| | C (%) | H (%) | N (%) |
|---|---|---|---|
| Calculé : | 64,84 | 7,07 | 15,12 |
| Trouvé : | 64,98 | 7,16 | 15,14 |

et

- le N-[3-(4-diméthylaminométhylpyrid-2-yloxy)propyl]3-pyridinecarboxamide 1-oxyde ; SR 58098 ; F. 103-106°C (Exemple 21) ; Rendement : 35 % de la théorie, identique au produit de l'exemple 19.

EXEMPLES 22 à 39.

En opérant comme décrit à l'exemple 16, par réaction de 0,03 mol de 3-(3-butylaminométhylphénoxy)propylamine, de 3-(3-allylaminométhylphénoxy)propylamine, de 3-(3-propargylaminométhylphénoxy)propylamine, de 3-[(2-hydroxyéthyl)aminométhylphénoxy]propylamine, de 3-(3-benzylaminométhylphénoxy)propylamine, de 3-(3-cyclopropylméthylaminométhylphénoxy)propylamine, de 3-(3-phénylaminométhylphénoxy)propylamine, de 3-(3-cyclopentylaminométhylphénoxy)propylamine, de 3-(3-diallylaminométhylphénoxy)propylamine, de 3-(3-dipropargylaminométhylphénoxy)propylamine, de 3-[3-di(2-hydroxyéthyl)aminométhylphénoxy]propylamine, de 3-[3-di(2-méthoxyéthyl)aminométhylphénoxy]propylamine, de 3-[(méthylcyclopropylméthyl)aminométhylphénoxy]propylamine, de 3-[4-(2-hydroxyéthyl-

pipérazino)méthylphénoxy]propylamine, de 3-(3,4,5-triméthyl-pipérazinométhylphénoxy)propylamine, de 3-(3,4-diméthylpipérazino-méthylphénoxy)propylamine, de 3-(3,5-diméthylpipéridinométhyl-phénoxy)propylamine et, respectivement, de 3-[4-(2-hydroxyéthyl)-3-méthylpipérazinométhylphénoxy]propylamine avec 0,03 mol de 3-pyridinecarboxylate de 4-nitrophényle 1-oxyde on obtient, respectivement,

- le N-[3-(3-butylaminométhylphénoxy)propyl]-3-pyridinecarboxamide 1-oxyde (Exemple 22) ;
- le N-[3-(3-allylaminométhylphénoxy)propyl]-3-pyridinecarboxamide 1-oxyde (Exemple 23) ;
- le N-[3-(3-propargylaminométhylphénoxy)propyl]-3-pyridinecarboxamide 1-oxyde (Exemple 24) ;
- le N-[3-[(2-hydroxyéthyl)aminométhylphénoxy]propyl]-3-pyridine-carboxamide 1-oxyde (Exemple 25) ;
- le N-[3-(3-benzylaminométhylphénoxy)propyl]-3-pyridinecarboxamide 1-oxyde (Exemple 26) ;
- le N-[3-(3-cyclopropylméthylaminométhylphénoxy)propyl]-3-pyridine-carboxamide 1-oxyde (Exemple 27) ;
- le N-[3-(3-phénylaminométhylphénoxy)propyl]-3-pyridinecarboxamide 1-oxyde (Exemple 28) ;
- le N-[3-(3-cyclopentylaminométhylphénoxy)propyl]-3-pyridine-carboxamide 1-oxyde (Exemple 29) ;
- le N-[3-(3-diallylaminométhylphénoxy)propyl]-3-pyridinecarboxamide 1-oxyde (Exemple 30) ;
- le N-[3-(3-dipropargylaminométhylphénoxy)propyl]-3-pyridine-carboxamide 1-oxyde (Exemple 31) ;
- le N-[3-[3-di(2-hydroxyéthyl)aminométhylphénoxy]propyl]-3-pyridine-carboxamide 1-oxyde (Exemple 32) ;
- le N-[3-[3-di(2-méthoxyéthyl)aminométhylphénoxy]propyl]-3-pyridine-carboxamide 1-oxyde (Exemple 33) ;
- le N-[3-[(méthyl-cyclopropylméthyl)aminométhylphénoxy]-propyl]-3-pyridinecarboxamide 1-oxyde (Exemple 34) ;
- le N-[3-[4-(2-hydroxyéthylpipérazino)méthylphénoxy]-propyl]-3-pyridinecarboxamide 1-oxyde (Exemple 35) ;
- le N-[3-(3,4,5-triméthylpipérazinométhylphénoxy)propyl]-3-pyridine-carboxamide 1-oxyde (Exemple 36) ;

- le N-[3-(3,4-diméthylpipérazinométhylphénoxy)propyl]-3-pyridinecarboxamide 1-oxyde (Exemple 37) ;
- le N-[3-(3,5-diméthylpipéridinométhylphénoxy)propyl]-3-pyridinecarboxamide 1-oxyde (Exemple 33) ; et respectivement
- le N-[3-[4-(2-hydroxyéthyl)-3-méthylpipérazinométhylphénoxy]propyl]-3-pyridinecarboxamide 1-oxyde (Exemple 39).

EXEMPLE 40.

Comprimés à base d'un des composés décrits aux exemples 1 à 12 et 18 à 39, ayant la composition suivante :

| | |
|---|---|
| principe actif | 150 mg |
| cellulose microcristalline | 75 mg |
| talc | 15 mg |
| polyvinylpyrrolidone | 30 mg |
| silice précipitée | 25 mg |
| stéarate de magnésium | 5 mg |

On mélange intimement dans une mélangeuse-pétrisseuse tous les ingrédients, sauf le lubrifiant, pendant 15 minutes, puis on pétrit par addition graduelle d'eau. On fait passer la masse à travers un tamis de 1,25 mm. On sèche le granulé dans une étuve à ventilation forcée jusqu'à ce qu'on obtienne une humidité résiduelle relativement réduite (environ 2 %). On uniformise le granulé, on ajoute le lubrifiant et l'on forme des comprimés par compression. Poids d'un comprimé : 300 mg.

De la même façon on prépare des comprimés contenant 250 mg de principe actif.

## REVENDICATIONS

1. Nicotinamide 1-oxyde N-substitué de formule

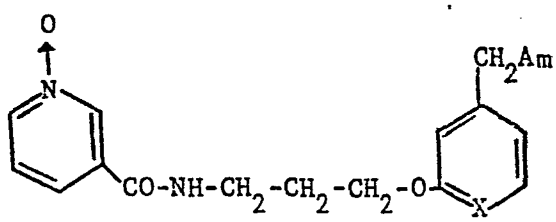

dans laquelle X représente un atome d'azote ou un groupe CH ; Am représente un groupe amino substitué par un alkyle inférieur, par un alkyle inférieur portant un groupe hydroxy, phényle ou cycloalkyle ayant 3 à 6 atomes de carbone ou par un radical choisi parmi alkényle inférieur, alkynyle inférieur, phényle et cycloalkyle ayant de 3 à 6 atomes de carbone ; un groupe amino substitué par deux groupes alkényle inférieur ou alkynyle inférieur, identiques ou différents ; un groupe amino substitué par deux groupes (hydroxy)alkyle inférieur ou (alkoxy inférieur)alkyle inférieur, identiques ou différents ; un groupe dialkylamino inférieur ; un groupe dialkylamino inférieur substitué par un groupe phényle, cycloalkyle ayant 3 à 6 atomes de carbone, méthoxyle ou hydroxyle ; un groupe pyrrolidino ; un groupe morpholino ; un groupe thiomorpholino ; un groupe hexaméthylèneimino ; un groupe pipéridino ; un groupe pipéridino substitué par un groupe méthyle, hydroxyle ou hydroxyméthyle ou par deux groupes méthyle ; un groupe pipérazino substitué en position 4 par un groupe alkyle inférieur ou (hydroxy)alkyle inférieur ; ou un groupe pipérazino substitué en position 4 par un groupe alkyle inférieur ou (hydroxy)-alkyle inférieur et portant aussi un groupe méthyle en positions 3 et/ou 5 ; ainsi que ses sels pharmaceutiquement acceptables.

2. N-[3-[3-(3-méthylpipéridinométhyl)phénoxy]propyl]-3-pyridinecarboxamide 1-oxyde et ses sels pharmaceutiquement acceptables.

3. N-[3-[3-(4-méthylpipéridinométhyl)phénoxy]propyl]-3-pyridinecarboxamide 1-oxyde et ses sels pharmaceutiquement acceptables.

4. N-[3-[3-(4-hydroxypipéridinométhyl)phénoxy]propyl]-3-pyridinecarboxamide 1-oxyde et ses sels pharmaceutiquement acceptables.

5. N-[3-(3-hexaméthylèneiminométhylphénoxy)propyl]-3-pyridinecarboxamide 1-oxyde et ses sels pharmaceutiquement acceptables.

6. Procédé pour la préparation de composés de formule

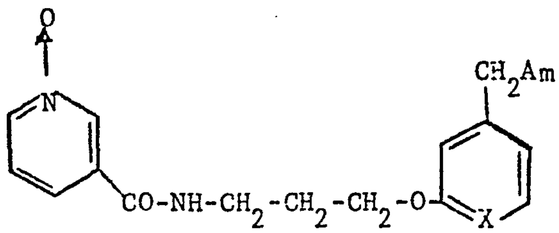

dans laquelle X représente un atome d'azote ou un groupe CH ; Am représente un groupe amino substitué par un alkyle inférieur, par un alkyle inférieur portant un groupe hydroxy, phényle ou cycloalkyle ayant 3 à 6 atomes de carbone ou par un radical choisi parmi alkényle inférieur, alkynyle inférieur, phényle et cycloalkyle ayant 3 à 6 atomes de carbone ; un groupe amino substitué par deux groupes alkényle inférieur ou alkynyle inférieur, indentiques ou différents ; un groupe amino substitué par deux groupes (hydroxy)alkyle inférieur ou (alkoxy inférieur)alkyle inférieur, identiques ou différents ; un groupe dialkylamino inférieur ; un groupe dialkylamino inférieur substitué par un groupe phényle, cycloalkyle ayant 3 à 6 atomes de carbone, méthoxyle ou hydroxyle ; un groupe pyrrolidino ; un groupe morpholino ; un groupe thiomorpholino ; un groupe hexaméthylèneimino ; un groupe pipéridino ; un groupe pipéridino substitué par un groupe méthyle, hydroxyle ou hydroxyméthyle ou par deux groupes méthyle ; un groupe pipérazino substitué en position 4 par un groupe alkyle inférieur ou (hydroxy)alkyle inférieur ; ou un groupe pipérazino substitué en position 4 par un groupe alkyle inférieur ou (hydroxy)alkyle inférieur et portant aussi 1 ou 2 groupes méthyle dans les positions 3 et 5 ; et de leurs sels pharmaceutiquement acceptables, caractérisé en ce qu'on traite une amine de formule

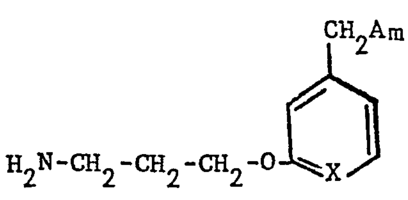

dans laquelle X et Am sont tels que définis ci-dessus, avec un dérivé fonctionnel de l'acide carboxylique de formule

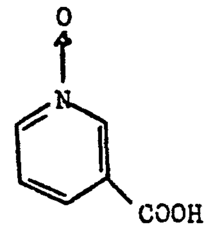

dans un solvant organique à une température comprise entre 0°C et la température d'ébullition du solvant employé et l'on transforme éventuellement le produit ainsi obtenu dans ses sels pharmaceutiquement acceptables.

7. Procédé selon la revendication 6, caractérisé en ce que comme dérivé fonctionnel on utilise le chlorure, sous forme de chlorhydrate.

8. Procédé selon la revendication 6, caractérisé en ce que le dérivé fonctionnel a la formule

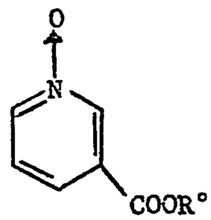

dans laquelle R° représente un groupe nitrophényle, méthoxyphényle, trityle, benzhydryle.

9. Procédé selon une des revendications 6 à 8, caractérisé en ce qu'on isole le produit final sous forme d'un de ses sels et l'on libère la base libre dudit sel par neutralisation à l'aide d'un hydroxyde ou carbonate alcalin.

10. Composition pharmaceutique renfermant, en tant que principe actif, un composé selon une des revendications 1 à 5.

11. Composition pharmaceutique sous forme d'unité de dosage à action $H_2$-bloquante renfermant de 10 à 1 000 mg d'un produit selon une des revendications 1 à 5 en mélange avec un excipient pharmaceutique.

12. Composition selon la revendication 11 renfermant de 50 à 500 mg d'un produit selon une des revendications 1 à 5.

## REVENDICATIONS

1. Procédé pour la préparation de composés de formule

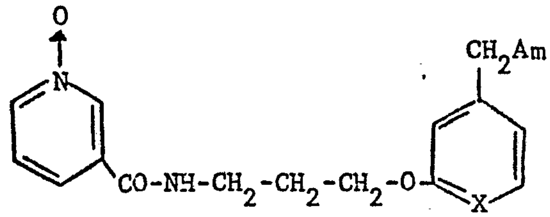

dans laquelle X représente un atome d'azote ou un groupe CH ; Am représente un groupe amino substitué par un alkyle inférieur, par un alkyle inférieur portant un groupe hydroxy, phényle ou cycloalkyle ayant 3 à 6 atomes de carbone ou par un radical choisi parmi alkényle inférieur, alkynyle inférieur, phényle et cycloalkyle ayant 3 à 6 atomes de carbone ; un groupe amino substitué par deux groupes alkényle inférieur ou alkynyle inférieur, identiques ou différents ; un groupe amino substitué par deux groupes (hydroxy)alkyle inférieur ou (alkoxy inférieur)alkyle inférieur, identiques ou différents ; un groupe dialkylamino inférieur ; un groupe dialkylamino inférieur substitué par un groupe phényle, cycloalkyle ayant 3 à 6 atomes de carbone, méthoxyle ou hydroxyle ; un groupe pyrrolidino ; un groupe morpholino ; un groupe thiomorpholino ; un groupe hexaméthylèneimino ; un groupe pipéridino ; un groupe pipéridino substitué par un groupe méthyle, hydroxyle ou hydroxyméthyle ou par deux groupes méthyle ; un groupe pipérazino substitué en position 4 par un groupe alkyle inférieur ou (hydroxy)alkyle inférieur ; ou un groupe pipérazino substitué en position 4 par un groupe alkyle inférieur ou (hydroxy)-alkyle inférieur, portant aussi un groupe méthyle en positions 3 et/ou 5 et de leurs sels pharmaceutiquement acceptables, caractérisé en ce qu'on traite une amine de formule

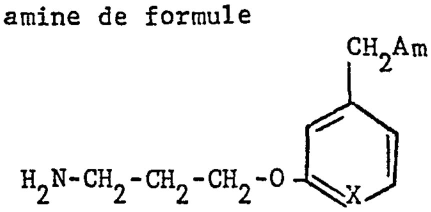

dans laquelle X et Am sont tels que définis ci-dessus, avec un dérivé fonctionnel de l'acide carboxylique de formule

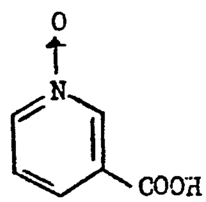

dans un solvant organique à une température comprise entre 0°C et la température d'ébullition du solvant employé et l'on transforme éventuellement le produit ainsi obtenu dans ses sels pharmaceutiquement acceptables.

2. Procédé selon la revendication 1, caractérisé en ce que comme dérivé fonctionnel on utilise le chlorure, sous forme de chlorhydrate.

3. Procédé selon la revendication 1, caractérisé en ce que le dérivé fonctionnel a la formule

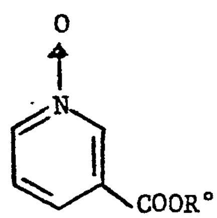

dans laquelle R° représente un groupe nitrophényle, méthoxyphényle, trityle, benzhydryle.

4. Procédé selon une des revendications 1 à 3, caractérisé en ce qu'on isole le produit final sous forme d'un de ses sels et l'on libère la base libre dudit sel par neutralisation à l'aide d'un hydroxyde ou carbonate alcalin.